# FASCICULE DE BREVET EUROPEEN

(11) **EP 1 596 776 B1**
(45) Date de publication et mention de la délivrance du brevet: **23.01.2008**
(21) Numéro de dépôt: 04710890.7
(22) Date de dépôt: 13.02.2004
(51) Int. Cl.: A61F 2/44

(54) **PROTHESE INTERVERTEBRALE**
INTERVERTEBRALE PROTHESE
INTERVERTEBRAL PROSTHESIS

(30) Priorité: 13.02.2003 FR 0301753
(43) Date de publication de la demande: 23.11.2005
(73) Titulaire: Spinevision, 75012 Paris (FR)
(72) Inventeur: PETIT, Dominique, F-62180 Verton (FR); DROULOUT, Thomas, F-78400 CHATOU (FR); SENE, Vincent, F-94230 Cachan (FR)
(74) Mandataire: Bredema
(86) Numéro de dépôt international: PCT/FR2004/000341
(87) Numéro de publication internationale: WO 2004/073561

(56) Documents cités:
- WO-A-01/68003
- DD-A- 239 523
- US-A- 5 674 294

## Description

La présente invention se rapporte au domaine des prothèses de disques intervertébraux.

La présente invention se rapporte plus particulièrement à une prothèse intervertébrale du type constituée au moins d'un noyau positionné entre un plateau supérieur et un plateau inférieur, ladite prothèse comportant en outre au moins une enveloppe externe.

Le document WO 0168003 A représente l'état de la technique le plus proche et définit le préambule de la revendication 1.

L'art antérieur connaît déjà des prothèses intervertébrales de ce type.

Il est ainsi proposé dans le brevet américain N° 5 674 296, une endoprothèse de disque vertébral comprenant un noyau, de préférence un noyau élastique, des éléments rigides ayant des surfaces internes concaves entourant au moins en partie le noyau. L'endoprothèse comprend aussi un élément d'étanchéité formé de matériau flexible, fixé aux éléments rigides et entourant le noyau.

Cette endoprothèse vertébrale de disque (18) comporte un noyau flexible (20, 24), des plateaux concaves-convexes rigides (30, 32, 34) disposés au-dessus et en dessous du noyau, un anneau de garniture périphérique (22) relativement rigide entourant le noyau (20, 24), et un membre de joint (110) constitué d'un matériau flexible, attaché aux plateaux concaves-convexes et entourant le noyau et l'anneau de garniture.

Chaque plateau concave-convexe est d'épaisseur en coupe relativement constante et présente d'une part une surface convexe externe pour s'engager dans la structure adjacente d'os qui a été préalablement fraisée et d'autre part une surface concave intérieure correspondante pour retenir le corps résilient du noyau.

Dans cette prothèse, la forme des faces intérieures des plateaux est identique à la forme du noyau adjacent et le noyau est ainsi strictement immobile.

Les prothèses de l'art antérieur ne donnent pas satisfaction car elles ne permettent pas de guider et d'amortir les déplacements des vertèbres sous-jacentes et sus-jacentes, comme le fond les disques anatomiques.

La présente invention entend remédier aux inconvénients de l'art antérieur en proposant une prothèse qui à la fois guide et amorti les mouvements de flexion/extension avant/arrière et d'inclinaison gauche/droite des vertèbres sous-jacentes et sus-jacentes.

Pour ce faire, la présente invention est comme définie par le revendication 1 et elle est remarquable, dans son acception la plus large, en ce que lesdits plateaux comportent respectivement des faces intérieures qui permettent un mouvement du noyau à l'intérieur de ladite prothèse.

Ledit noyau est, de préférence, entouré latéralement d'une enveloppe interne souple, ladite enveloppe interne présentant des directions privilégiées de flexibilité, de préférence au nombre de deux et orientées perpendiculairement.

Ladite enveloppe externe est, de préférence, souple.

De préférence au moins une face intérieure, respectivement desdits plateaux supérieur et/ou inférieur et de manière encore plus préférée les deux faces intérieures des plateaux, présente(nt) des moyens de guidage de déplacement dudit noyau.

Lesdits moyens de guidage sont, de préférence, orientés selon deux directions perpendiculaires et de préférence dans les mêmes directions que les directions privilégiées de flexibilité de l'enveloppe interne.

Lesdits moyens de guidages sont, de préférence, constitués par des plans inclinés dont les bords extérieurs sont orientés vers le noyau.

Dans une variante, au moins une face intérieure, respectivement desdits plateaux supérieur et/ou inférieur présente, de préférence, un appendice et ledit noyau comporte, de préférence, en outre respectivement sur sa face supérieure et/ou inférieure deux rainures orientées selon deux directions perpendiculaires.

Dans cette variante, lesdites rainures sont, de préférence, orientées dans les mêmes directions que les directions privilégiées de flexibilité de l'enveloppe interne.

Dans une variante, au moins une face intérieure, respectivement desdits plateaux supérieur et/ou inférieur, et de préférence les deux faces intérieures des plateaux, est ou sont plate(s).

Ledit noyau présente, de préférence, une forme sensiblement parallélépipédique.

Les faces supérieure et inférieure dudit noyau sont, de préférence, arrondies selon les directions privilégiées de flexibilité de l'enveloppe interne.

Ledit noyau présente, de préférence, des bords arrondis.

Ladite enveloppe interne présente, de préférence, en coupe horizontale une forme de croix constituée de quatre bras horizontaux orientés perpendiculairement.

Lesdits bras présentent, de préférence, chacun un trou débouchant sur les faces supérieure et inférieure de ladite enveloppe interne.

Les faces centripètes desdits bras sont, de préférence, droites et les faces centrifuges desdits bras sont, de préférence, arrondies.

Ladite enveloppe externe présente, de préférence, des ailettes intérieures destinées à maintenir les bras horizontaux de l'enveloppe interne, à l'angle entre les bras de la forme en croix.

Dans une variante, l'enveloppe interne et l'enveloppe externe sont venues de matière.

Ladite enveloppe externe est, de préférence, ajourée sur ses parois supérieure et/ou inférieure, pour le passage des faces extérieures, respectivement des plateaux supérieur et inférieur.

Dans une autre variante, ledit ou lesdits plateau(x) supérieur et/ou inférieur présente(nt) une cavité annulaire adjacente à la face extérieure et ladite ou lesdites paroi(s) respectivement supérieure et/ou inférieure présente(nt) un rebord centripète destiné à coopérer avec ladite cavité annulaire.

Dans une autre variante, ledit ou lesdits plateau(x) supérieur et/ou inférieur est ou sont souple(s).

Dans une autre variante, ledit ou lesdits plateau(x) supérieur et/ou inférieur présente(nt) sur sa ou sur leurs face(s) extérieure(s) un rail de fixation.

La prothèse comporte en outre, de préférence, des moyens de fixation pour la fixation respectivement du ou des plateau(x) supérieur et/ou inférieur.

Dans un mode de réalisation avantageux de l'invention, la prothèse intervertébrale comporte deux noyaux.

De préférence, l'enveloppe interne présente une membrane médiane longitudinale séparant les noyaux.

Avantageusement, la membrane de ladite enveloppe interne est flexible.

Avantageusement, un des plateaux au moins comporte un orifice traversant pour permettre le passage d'un fluide dans ladite enveloppe interne.

Avantageusement, l'orifice traversant est prolongé par une zone tubulaire filetée destinée à coopérer avec une zone filetée complémentaire d'un élément de contact destinée à être mis au contact avec une des vertèbres.

Avantageusement, ladite prothèse formée du ou des noyaux, de l'enveloppe interne, de l'enveloppe externe et des plateaux est surmoulé avec un élastomère souple.

Avantageusement, le(s)dit(s) noyau(x) est (sont) solide(s) ou liquide(s).

Avantageusement, la présente invention permet de restaurer les mouvements naturels des vertèbres sous-jacentes et sus-jacentes, ou, au besoin, limiter certaines capacités de mouvement dans une direction ou une autre.

Avantageusement également, il est possible de choisir des matériaux constitutifs en fonction des résistances croissantes au mouvement souhaitées et en fonction des directions privilégiées de flexibilité souhaitées pour chacune des pièces :
- le plateau supérieur et/ou inférieur, et/ou
- le noyau, et/ou
- l'enveloppe interne, et/ou
- l'enveloppe externe.

Avantageusement également, la présente invention permet une bonne absorption des chocs.

Elle permet en outre de préserver les facettes articulaires.

Elle présente une grande fiabilité dans le temps.

Elle est en outre simple à implanter.

On comprendra mieux l'invention à l'aide de la description, faite ci-après à titre purement explicatif, d'un mode de réalisation de l'invention, en référence aux figures annexées :
- la figure 1 illustre une vue en perspective de la version de base de la prothèse selon l'invention ;
- la figure 2 illustre une vue éclatée de la version de base de la prothèse selon l'invention;
- la figure 3 illustre une vue en coupe verticale selon AA de la figure 1 ;
- la figure 4 illustre une vue en perspective de la coupe horizontale partielle selon BB de la figure 1 ;
- la figure 5 illustre une vue de dessus de la coupe horizontale partielle selon BB de la figure 1 ;
- la figure 6 illustre une vue de dessus de la version de base de la prothèse selon l'invention, sans le plateau supérieur ;
- la figure 7 illustre une vue en coupe verticale selon AA de la figure 1 de l'enveloppe externe de la version de base de la prothèse selon l'invention ;
- la figure 8 illustre une vue en perspective d'un plateau pour la version de base de la prothèse selon l'invention ;
- la figure 9 illustre une vue en coupe verticale selon AA de la figure 1 du noyau de la version de base de la prothèse selon l'invention ;
- la figure 10 illustre une vue en coupe transversale du noyau de la version de base de la prothèse selon l'invention ;
- la figure 11 illustre une vue en coupe verticale selon AA de la figure 1 de l'enveloppe interne de la version de base de la prothèse selon l'invention ;
- la figure 12 illustre une vue en coupe verticale selon AA de la figure 1 de la version de base de la prothèse selon l'invention lors d'un mouvement de flexion ;
- la figure 13 illustre la déformation de l'enveloppe externe lors du mouvement illustré figure 12 ;
- la figure 14 illustre une vue en coupe verticale et en perspective d'une variante de l'invention dans laquelle les plateaux supérieur et inférieur sont clipés dans l'enveloppe externe ;
- la figure 15 illustre une vue en perspective d'un plateau pour la variante illustrée figure 14;
- la figure 16 illustre une vue éclatée d'une variante de l'invention dans laquelle le noyau présente une forme de parallélépipède droit et dans laquelle l'enveloppe interne est constituée de quatre bras séparés ;
- la figure 17 est une vue en coupe verticale et en perspective de la variante de la figure 16 assemblée ;
- la figure 18 est une vue en coupe horizontale partielle et en perspective de la variante de la figure 16 assemblée ;
- la figure 19 illustre une vue en coupe verticale de la variante de la figure 16 lors d'un mouvement de flexion ;
- la figure 20 illustre une vue éclatée d'une variante de l'invention dans laquelle le noyau présente une forme de parallélépipède à faces supérieur et inférieur arrondie et munies de rainures, dans laquelle les plateaux supérieur et inférieur sont munis d'un appendice destiné à coopérer avec lesdites rainures et dans laquelle l'enveloppe interne est constituée de quatre bras séparés ;
- la figure 21 est une vue en coupe verticale et en perspective de la variante de la figure 20 assemblée;
- la figure 22 est une vue en coupe horizontale partielle et en perspective de la variante de la figure 20 assemblée;
- la figure 23 illustre une vue en perspective du noyau de la variante de la figure 20;
- la figure 24 illustre une vue en coupe longitudinale du noyau pour la variante de la figure 20 ;
- la figure 25 illustre une vue en perspective d'un plateau pour la variante de la figure 20 ;
- la figure 26 illustre une vue en perspective d'une variante de l'invention dans laquelle les plateaux supérieur et inférieur sont munis d'un rail de fixation ;
- la figure 27 illustre une vue de côté de la variante de la figure 26 ;
- la figure 28 illustre une vue en perspective d'un plateau pour la variante de la figure 26 ;
- la figure 29 illustre une vue en perspective d'une prothèse selon la variante de la figure 26 munie de moyens de fixation pour sa fixation aux vertèbres ; et
- la figure 30 illustre une vue éclatée d'une autre variante de la prothèse intervertébrale selon l'invention ;
- la figure 31 illustre une vue en coupe verticale en en perspective de la prothèse de la figure 30 assemblée ;
- la figure 32 illustre une vue de dessus et en perspective d'un plateau constituant la prothèse de la figure 30 ; et
- la figure 33 illustre une vue en coupe et en perspective du plateau de la figure 32.

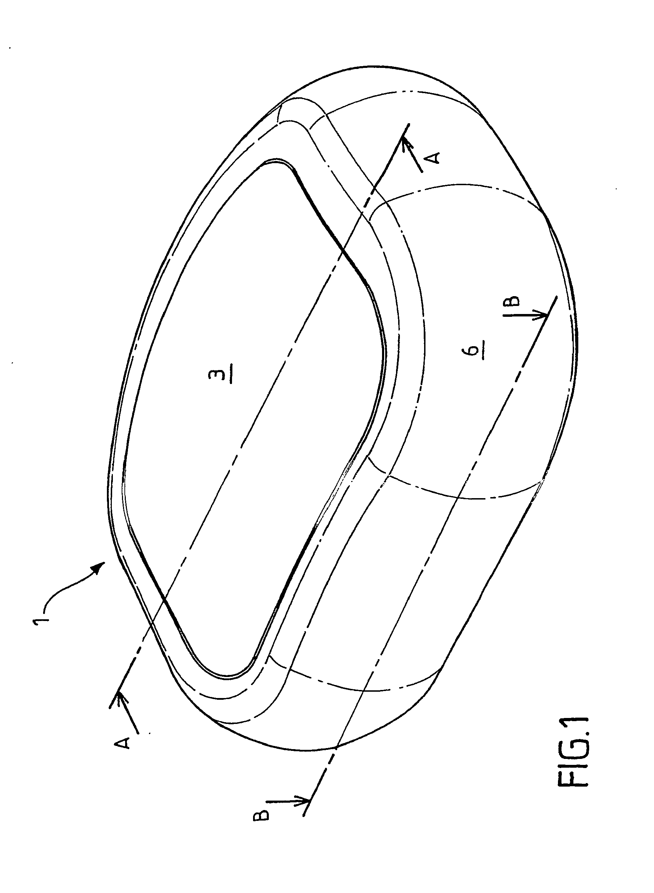

La prothèse selon la présente invention, illustrée dans sa version de base sur la figure 1, est une prothèse intervertébrale (1) du type constituée au moins d'un noyau (2) positionné entre un plateau supérieur (3) et un plateau inférieur (4).

Dans cette version de base les plateaux (3, 4) sont rigides.

Cette prothèse est destinée à être positionnée entre deux vertèbres, à la place d'un disque, lorsque celui-ci est défaillant et provoque des douleurs.

Les plateaux supérieur (3) et inférieur (4) présentent respectivement une face intérieure (31, 41).

Le plateau supérieur (3) présente une face extérieure (32) destinée à coopérer avec la face inférieure de la vertèbre située au-dessus de la prothèse et le plateau inférieur (4) présente une face extérieure (42) destinée à coopérer avec la face supérieure de la vertèbre située au-dessous de la prothèse. Les faces extérieures (32, 42) sont sensiblement horizontales.

Le but de la prothèse selon l'invention est de se substituer complètement au disque douloureux et de permettre à nouveau les mouvements des vertèbres situées au-dessus et en dessous, les unes par rapport aux autres, comme lorsque le disque n'était pas défaillant.

Pour autoriser ces mouvements des vertèbres, ledit noyau (2) est mobile à l'intérieur de la prothèse, entre les faces intérieures (31, 41) des plateaux (3, 4).

La mobilité du noyau (2) est essentiellement horizontale et engendre une modification du centre de gravité de la prothèse.

La forme des faces intérieures (31, 41) est complémentaire de la forme du noyau adjacent, sans être complètement identique, de manière à autoriser le déplacement du noyau (2) par rapport aux plateaux.

Le noyau (2) est entouré latéralement d'une enveloppe interne (5). Cette enveloppe interne (5) est souple et présente des directions privilégiées de flexibilité. Ladite prothèse (1) comporte en outre une enveloppe externe (6) également souple.

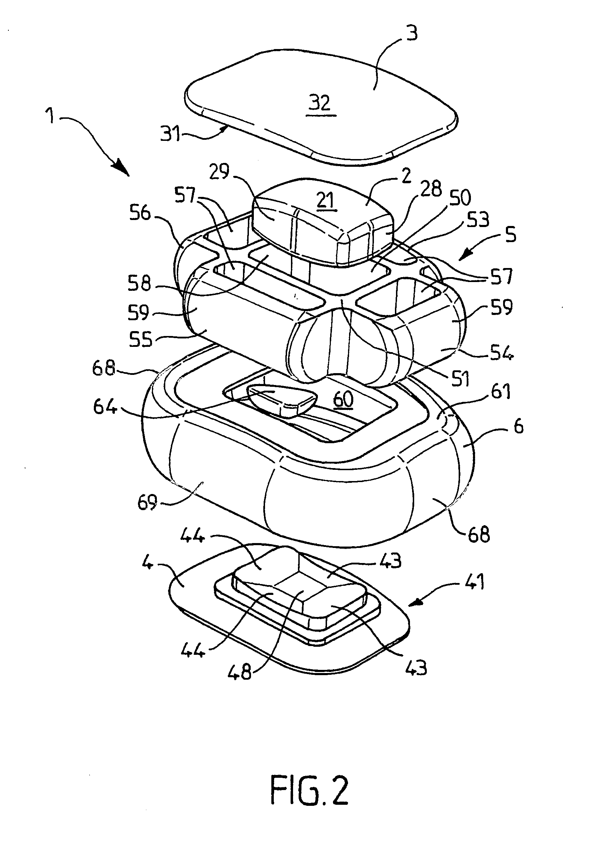

Cette enveloppe externe (6) contient l'enveloppe interne (5), qui elle-même contient le noyau (2), comme on peut le voir sur les figures 2 à 5.

Ladite enveloppe interne (5) présente deux directions privilégiées de flexibilité orientées perpendiculairement, afin de permettre de restaurer d'une part les mouvements d'inclinaison latérales gauche/droite et d'autre part les mouvements de flexion/extension, les mouvements de rotation étant restaurés notamment à l'intersection des deux directions privilégiés autorisant respectivement les inclinaisons gauche/droite et les mouvements de flexion/extension.

Les éléments souples de la prothèse selon l'invention peuvent être constitués d'éléments réalisés en bio matériau (polyuréthane, polycarbonate, polyvinyle alcool, ...) et les éléments rigides peuvent être constitués d'éléments réalisés en bio matériau ou en métal ou alliage métallique.

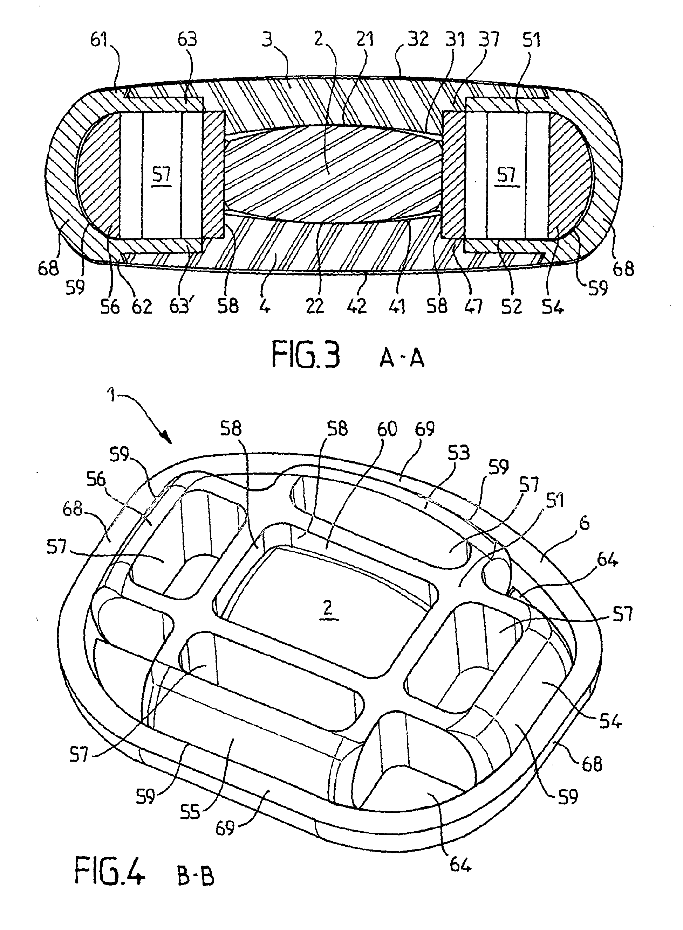

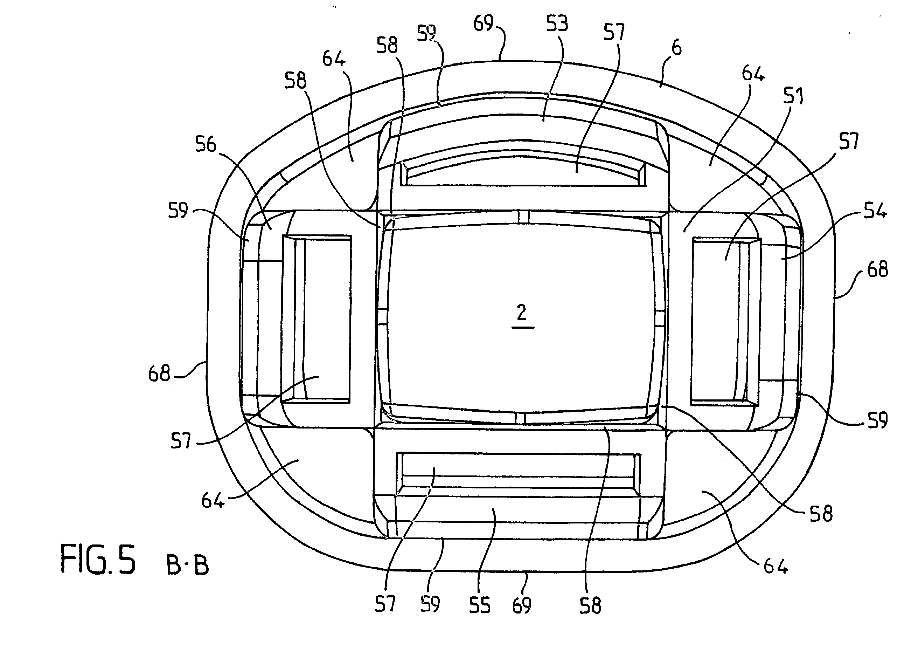

L'enveloppe externe (6) présente une forme générale parallélépipédique et comporte des parois supérieure (61), inférieure (62) et latérales frontales (68) et sagittales (69). Les parois latérales frontales (68) sont sensiblement arrondies selon un arc de cercle vertical, comme on peut le voir sur la figure 3, alors que les parois latérales sagittales (69) sont arrondies à la fois selon un arc de cercle horizontal et selon un arc de cercle vertical, comme on peut le voir sur les figures 5 à 7. Les parois supérieure (61) et inférieure (62) sont sensiblement plates.

Les angles entre les parois de l'enveloppe externe (6) sont arrondis selon un faible rayon de courbure.

L'enveloppe externe (6) présente une cavité intérieure (60) en forme de croix en coupe horizontale, comme on peut le voir sur la figure 5, dans laquelle est positionnée l'enveloppe interne (5).

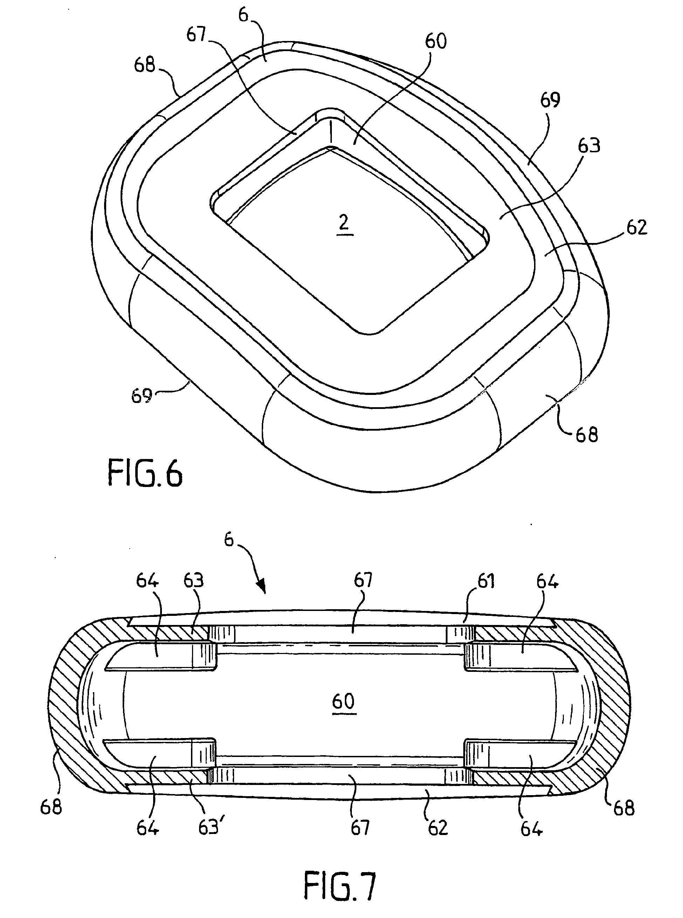

L'enveloppe externe (6) présente un trou (67) sur ses parois supérieure (61) et inférieure (62), comme on peut le voir sur la figure 6, pour le passage des faces extérieures (32, 42), respectivement des plateaux supérieur (3) et inférieur (4).

Les parois latérales frontales (68) et sagittales (69) présentent une épaisseur sensiblement constante.

Ladite paroi supérieure (61) ou inférieure (62), et de préférence les deux parois supérieure (61) et inférieure (62), présente(nt) respectivement un rebord centripète (63, 63'), ménagé vers l'intérieur, sur lequel/lesquels vient/viennent se reposer la/les face(s) extérieure(s) (32, 42) du/des plateau(x) supérieur (3) et/ou inférieur (4), comme on peut le voir sur la figure 3.
ladite enveloppe externe (6) présente des ailettes intérieures (64) horizontales, positionnées dans la partie inférieure et supérieure de la paroi intérieure de l'enveloppe et orientées vers l'intérieur.

La cavité intérieure (60) présente donc à la fois une forme de croix en coupe horizontale et une forme de croix en coupe verticale, comme on peut le constater sur la figure 7.

La flexibilité et la souplesse de l'enveloppe externe (6) permettent de maintenir l'ensemble des éléments constitutifs de la prothèse et participent à l'amortissement vertical, c'est-à-dire à la résistance à la compression.

Cette flexibilité et cette souplesse de l'enveloppe externe (6) permettent également de réaliser un amortissement et un retour à la position centrale lors des mouvements de rotation, afin d'apporter une limitation dans ces mouvements.

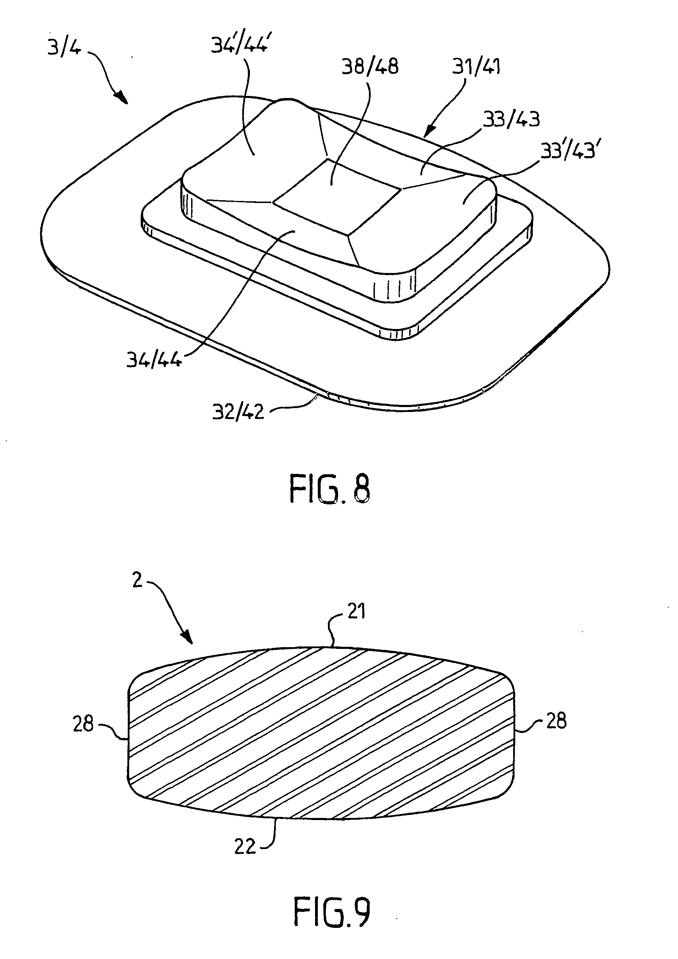

Comme illustré figure 8, les deux faces intérieures (31, 41) présentent des moyens de guidage de déplacement dudit noyau (2), lesdits moyens de guidage étant orientés selon deux directions perpendiculaires.

Ces moyens de guidages sont orientés dans les mêmes directions que les directions privilégiées de flexibilité de l'enveloppe interne (5).

Lesdits moyens de guidages sont constitués, pour le plateau supérieur (3) par des plans inclinés (33, 33', 34, 34') dont les bords extérieurs sont abaissés vers le noyau (2), c'est-à-dire vers le bas, et par un plan de fond (38) horizontal et pour le plateau inférieur (4) par des plans inclinés (43, 43', 44, 44') dont les bords extérieurs sont relevés vers le noyau (2), c'est-à-dire vers le haut, et par un plan de fond (48) horizontal.

Les plateaux supérieur (3) et inférieur (4) comportent en outre chacun un rebord (37, 47) rectangulaire, horizontal, situé entre la face intérieure (31, 41) et la face extérieure (32, 42), de taille supérieure à la face intérieure (31, 41) et de taille inférieure à la face extérieure (32, 42). Ces rebords (37, 47) sont destinés à être insérés dans les trous (67) ménagés dans les parois supérieure (61) et inférieure (62) de l'enveloppe externe (6).

Dans une variante de la version de base, un plateau, et de préférence les deux plateaux supérieur (3) et inférieur (4), est ou sont souple(s). Cette variante est avantageusement associée avec la variante de l'invention à noyau rigide.

Dans cette variante à plateaux souples, la souplesse des plateaux favorise l'amortissement vertical et augmente l'aptitude des plateaux à se conformer à la configuration des parois osseuses adjacentes.

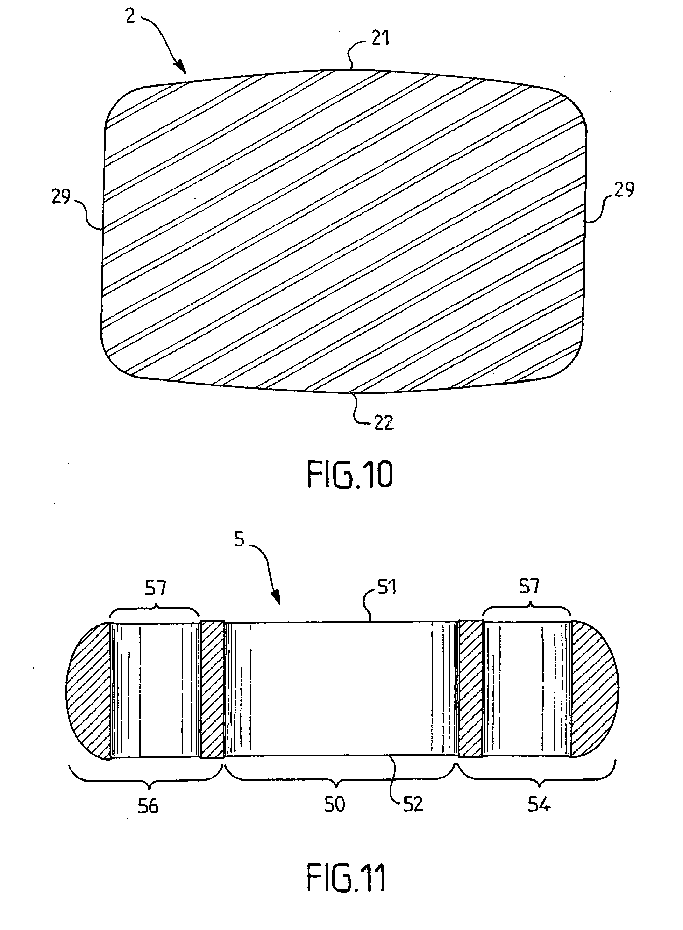

Le noyau (2), illustré figures 9 et 10, présente une forme sensiblement parallélépipédique et comporte des parois supérieure (21), inférieure (22) et latérales frontales (28) et sagittales (29). Les parois latérales frontales (28) et sagittales (29) sont sensiblement droites, comme on peut le voir sur les figures 2 et 9. Les parois supérieure (21), inférieure (22) sont arrondies selon deux arc de cercle verticaux perpendiculaires, comme on peut le voir sur ces mêmes figures.

Les faces supérieure (21) et inférieure (22) dudit noyau (2) sont arrondies selon les directions privilégiées de flexibilité de l'enveloppe interne (5).

Les angles ou bords entre les parois du noyau (2) sont arrondis selon un faible rayon de courbure.

L'enveloppe interne (5) présente une cavité centrale (50) dans laquelle est positionné le noyau (2).

Dans la version de base de l'invention, ledit noyau (2) est rigide.

Cette cavité (50) présente une forme de parallélépipède droit, de sorte que dans la version de base, des espaces vides sont ménagés aux extrémités frontales supérieure et inférieure, ainsi qu'aux extrémités sagittales supérieure et inférieure de cette cavité, entre la paroi de cette cavité et la paroi extérieure du noyau (2), comme on peut le constater sur la figure 3.

L'enveloppe interne (5) présente en coupe horizontale une forme de croix constituée de quatre bras horizontaux (53, 54, 55, 56) orientés perpendiculairement.

Ces bras (53, 54, 55, 56) s'étendent selon les directions privilégiées de flexibilité de l'enveloppe interne (5).

Dans la version de base de l'invention, les bras (53, 54, 55, 56) forment un tout unique, c'est-à-dire que l'enveloppe interne (5) constitue une seule pièce.

Les bras (53, 54, 55, 56) présent chacun un trou (57) débouchant sur les faces supérieure (51) et inférieure (52) de ladite enveloppe interne (5), comme on peut le voir sur les figures 4, 5 et 11, afin d'augmenter la flexibilité des bras. Ces trous sont vides ou remplis d'un fluide ou d'une matière élastique.

Les faces centripètes (58) desdits bras (53, 54, 55, 56), délimitant la cavité (50) sont droites.

Les faces centrifuges (59) desdits bras (53, 54, 55, 56) sont arrondies pour s'adapter aux faces intérieures des parois frontales (68) et sagittales (69) de l'enveloppe externe (6).

Les ailettes intérieures (64) de ladite enveloppe externe (6) sont ainsi destinées à maintenir les bras horizontaux (53, 54, 55, 56) de l'enveloppe interne (5), de manière à créer une rigidité supplémentaire dans les directions qui ne sont pas les directions privilégiées de flexibilité de l'enveloppe interne (5).

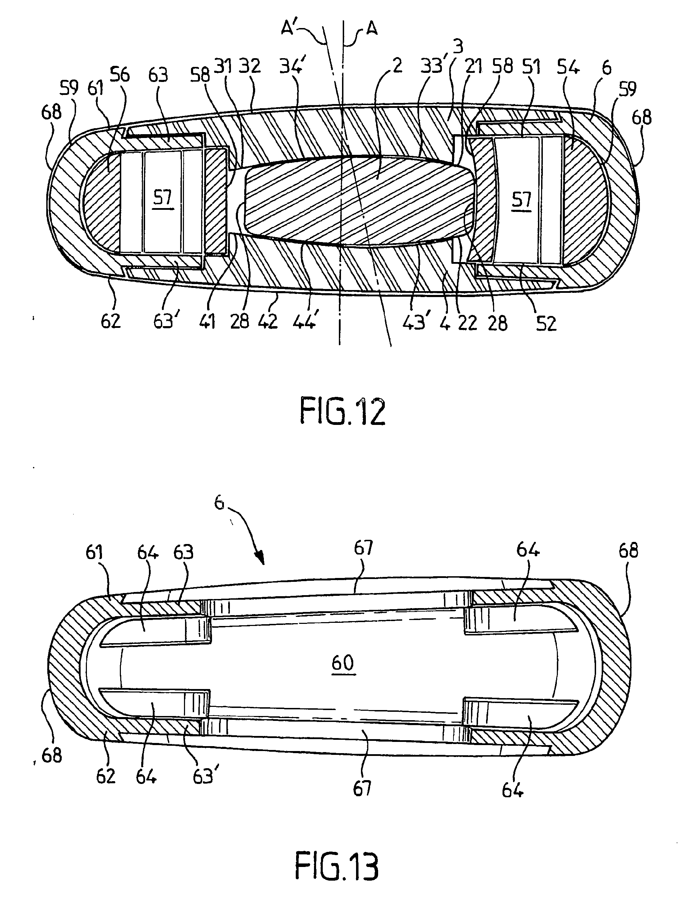

La flexibilité générale de la prothèse dans la version de base est importante dans les plans frontaux et sagittaux. En effet, lors de la flexion de la colonne vertébrale par exemple vers l'avant, comme illustré figure 12, il y a déplacement du noyau (2) dans l'enveloppe interne (5), vers l'arrière de la cavité (50) et une déformation de la face centripète (58) arrière de l'enveloppe interne (5). L'axe moyen de la colonne A au niveau de la prothèse, qui est passé de la position verticale à la position inclinée A' vers l'avant, a subi une rotation par rapport à la verticale et une translation vers l'arrière. Pendant ce mouvement de flexion vers l'avant, la face supérieure (21) du noyau (2) glisse sur les plans inclinés (33', 34') et la face inférieure (22) du noyau (2) glisse sur les plans inclinés (43', 44').

La face centripète (58) du bras (52) se déforme pour permettre le déplacement du noyau (2) relativement rigide et offre une force de résistance opposée qui est fonction de la flexibilité du matériau employé pour l'enveloppe interne (5). Plus le déplacement du noyau (2) est grand, plus la force opposée à ce déplacement est grande.

Grâce à la flexibilité de l'enveloppe extérieure (6), les plateaux (3, 4) s'écartent à l'arrière de la prothèse et se rapprochent à l'avant, selon la direction verticale. La figure 13 illustre la déformation de l'enveloppe extérieure (6) lors de ce même mouvement de flexion vers l'avant.

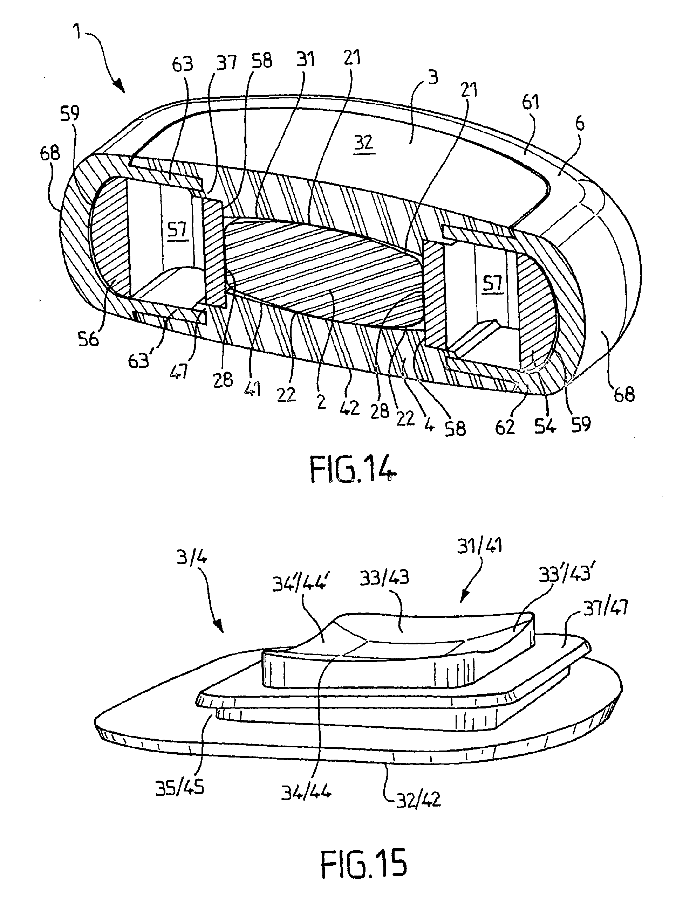

Dans une variante illustrée figures 14 et 15, le plateau supérieur (3) ou le plateau inférieur (4), et de préférence les deux plateau(x) supérieur (3) et inférieur (4), présente(nt) une cavité annulaire (35, 45) adjacente à la face extérieure (32, 42).

Cette cavité annulaire (35, 45), sensiblement rectangulaire, est destinée à coopérer avec le rebord centripète (63, 63') de l'enveloppe externe (6).

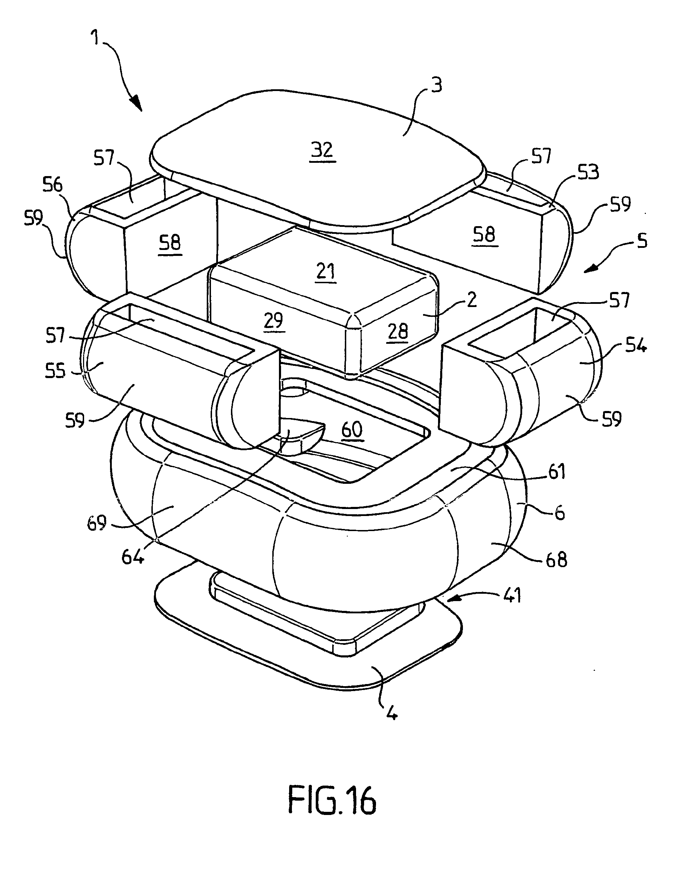

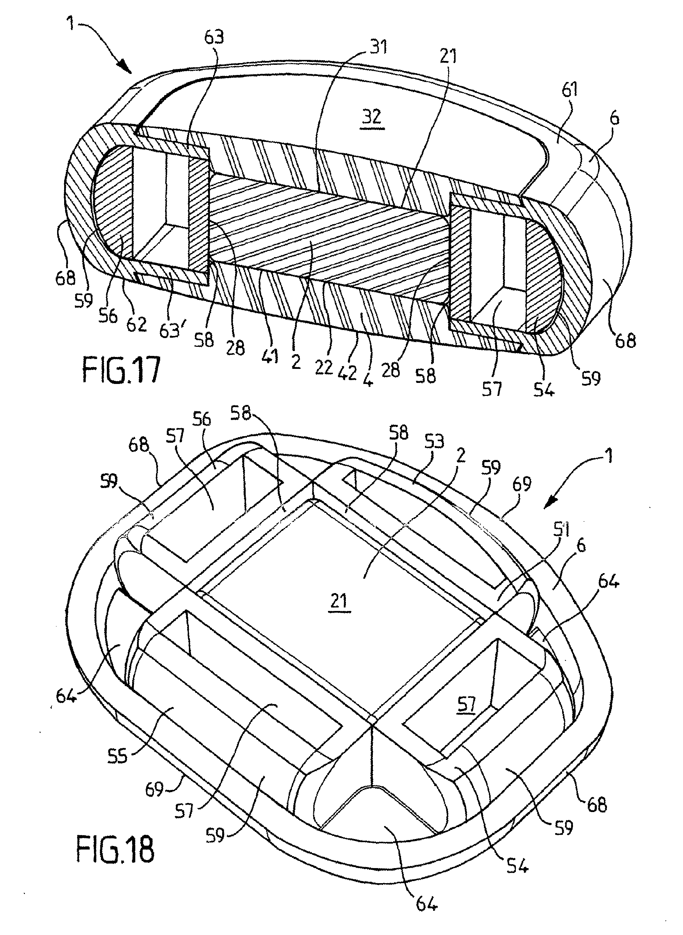

Dans une variante illustrée figures 16 à 19, le noyau (2) présente une forme sensiblement de parallélépipède droit. Les parois supérieure (21), inférieure (22) sont donc sensiblement horizontales et ne présentent pas de courbure ; Toutefois, les bords des parois présentent tout de même un arrondi.

Dans cette variante, le noyau (2) est plus souple que dans la version de base.

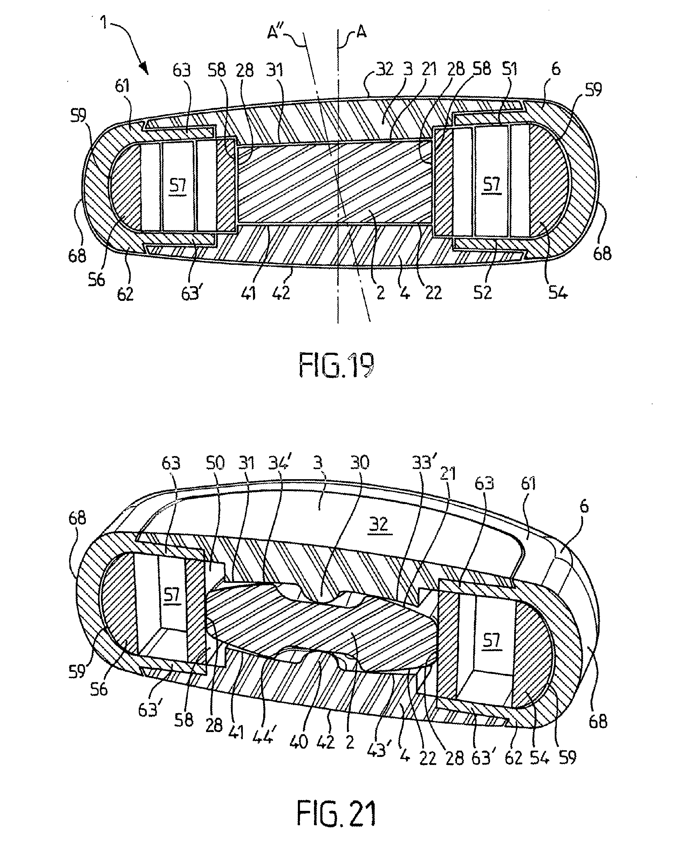

Dans cette variante, la flexibilité générale de la prothèse est amoindrie par rapport à celle de la prothèse selon la version de base. En effet, lors de la flexion vers l'avant, comme illustré figure 19, il n'y a pas de déplacement important du noyau (2) dans l'enveloppe interne (5), comme avec la version de base (cf. figure 12). L'axe moyen de la colonne A qui est passé de la position verticale à la position inclinée A'' vers l'avant n'a sensiblement subi qu'une rotation par rapport à la verticale.

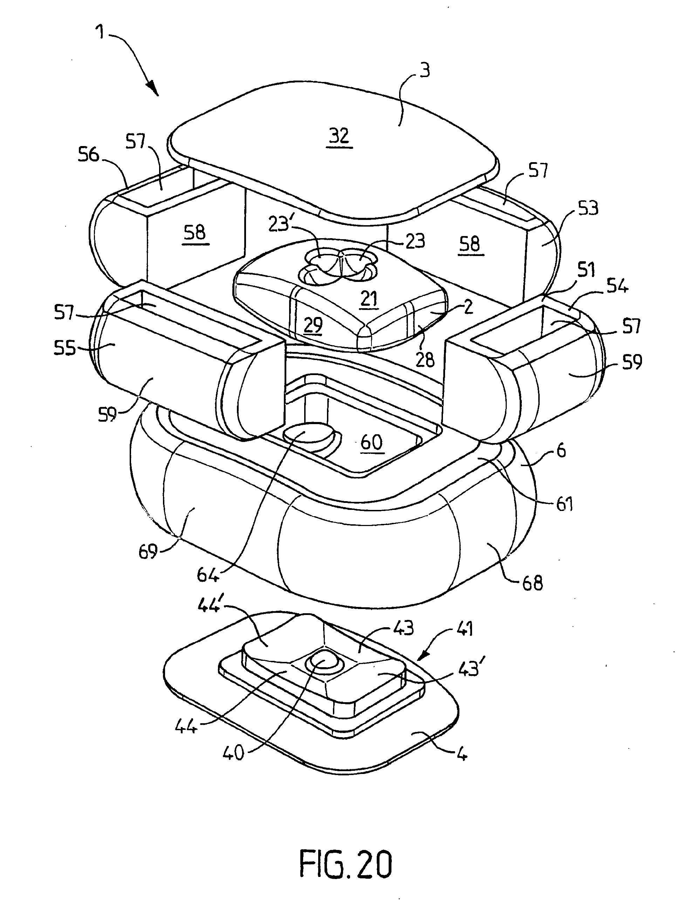

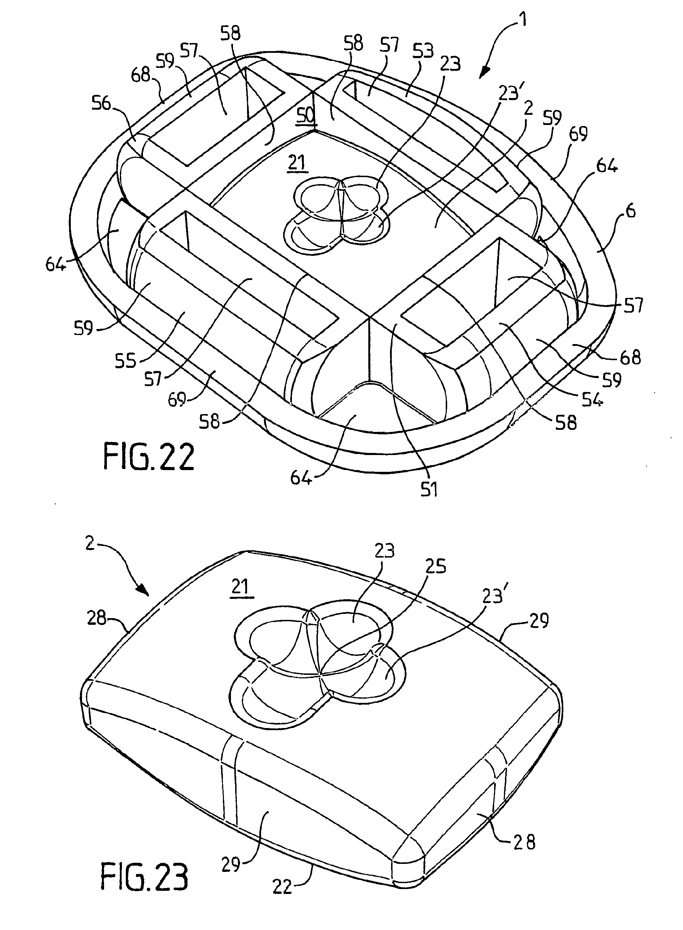

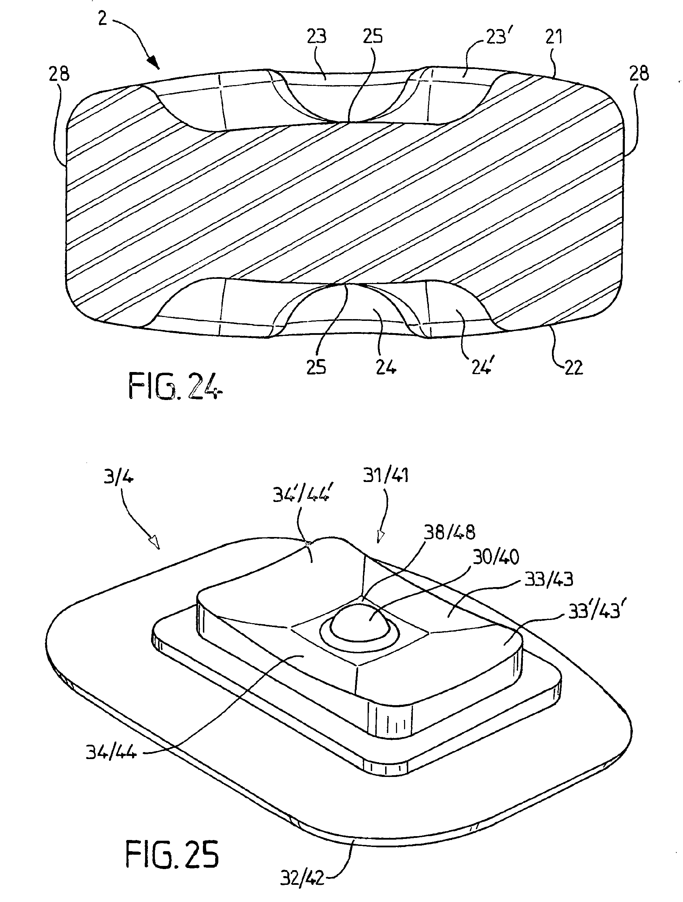

Dans une variante illustrée figures 20 à 25, au moins une face intérieure (31, 41), respectivement desdits plateaux supérieur (3) et/ou inférieur (4) présente un appendice (30, 40) constitué, par exemple, par une demi-sphère positionnée au centre du plan de fond (38, 48) de la face intérieure (31, 41) du plateau et orientée vers l'intérieur de la prothèse.

Il est à noter que cette variante est compatible avec les deux variantes suivantes: la variante dans laquelle les faces intérieures des plateaux sont plates et la variante dans laquelle les bords centrifuges extérieurs des faces intérieures des plateaux sont relevés vers le noyau (2).

De plus, dans cette variante, ledit noyau (2) comporte respectivement sur sa face supérieure (21) et/ou inférieure (22) deux rainures (23/23', 24/24') orientés selon deux directions perpendiculaires et destinées à coopérer respectivement avec l'appendice (30, 40). Ces rainures présentent en coupe transversale une forme semi-circulaire présentant un rayon sensiblement identique au rayon de la forme semi-sphérique de l'appendice (30, 40).

Lesdites rainures (23/23', 24/24') sont orientées dans les mêmes directions que les directions privilégiées de flexibilité de l'enveloppe interne (5), c'est-à-dire, d'une part dans la direction d'inclinaison latérale gauche/droite pour les rainures (23, 24) et d'autre part dans la direction de flexion/extension pour les rainures (23', 24').

La longueur de chaque rainure est adaptée en fonction de l'amplitude souhaitée pour chaque mouvement d'inclinaison latérale ou de flexion/extension.

Les extrémités des rainures sont arrondies selon le même rayon que le rayon de la forme semi-circulaire transversale et le point (25, 26) d'intersection respectivement des deux rainures (23, 23') sur la face supérieure (21) et des rainures (24, 24') sur la face inférieure (22) est aménagé pour permettre à l'appendice (30, 40) de se mouvoir dans n'importe quelle direction privilégiée ménagée par les rainures à partir de ce point d'intersection.

Dans cette variante en outre, les bras (53, 54, 55, 56) forment chacun un tout unique, c'est-à-dire que l'enveloppe interne (5) est constituée de quatre pièces : les quatre bras, comme on peut le voir sur la figure 20.

La forme en croix horizontale de l'enveloppe interne (5) est ainsi maintenue par l'enveloppe externe (6) périphérique.

Il est possible de prévoir que les bras ne présentent pas tous la même flexibilité. Il est, par exemple, possible de réaliser les bras arrière (54) et avant (56) plus souple que les bras gauche (53) et droit (55). Ainsi, la flexibilité de la prothèse est plus importante dans les mouvements de flexion/extension, que dans les mouvements d'inclinaison latérale.

Il est également possible d'imaginer que l'enveloppe interne (5) et l'enveloppe externe (6) forment une seule pièce, présentant éventuellement des caractéristiques intrinsèques non uniformes.

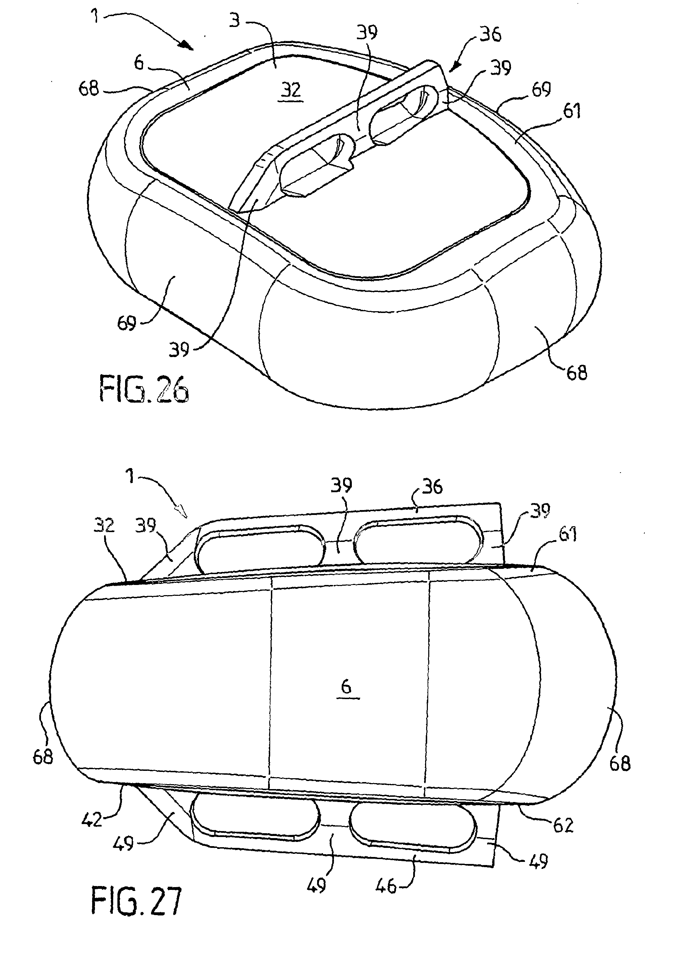

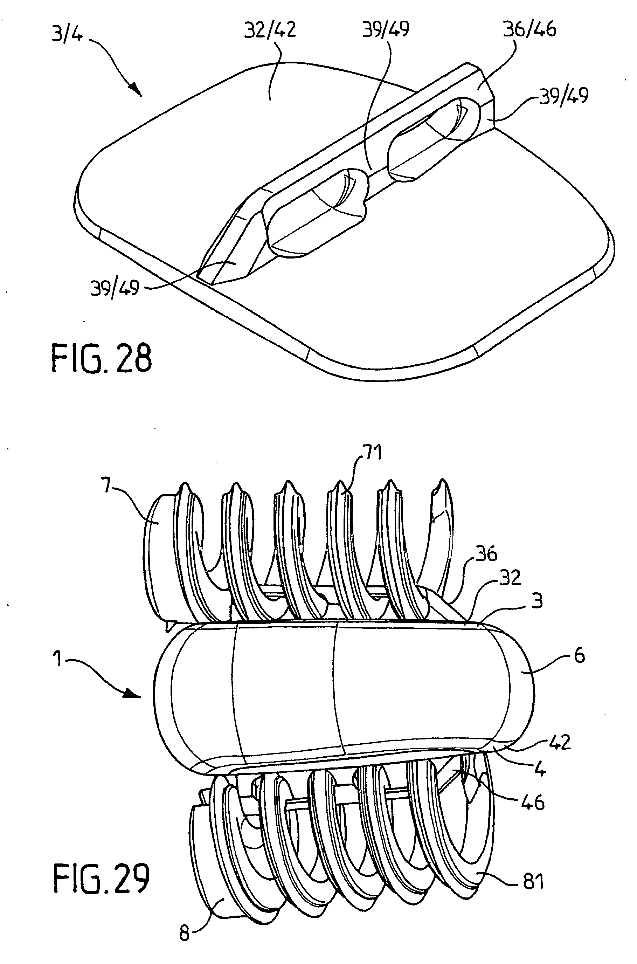

Dans une variante illustrée figures 26 à 29, ledit ou lesdits plateau(x) supérieur (3) et/ou inférieur (4) présente(nt) sur sa ou sur leur face(s) extérieure(s) (32, 42) un rail de fixation (36, 46).

Ce rail de fixation est orienté sensiblement selon la direction sagittale de flexion/extension. Il est constitué d'un pont longitudinal relié à la face extérieure (32, 42) du plateau (3, 4) par au moins un pilier (39, 49), et de préférence trois piliers (39, 49). Entre chaque pilier, une lumière est aménagée, afin de permettre de faire passer un moyen de fixation (7, 8).

De préférence, chaque moyen de fixation supérieur (7) et inférieur (8) est constitué d'une cage intersomatique du type de celle divulguée dans la demande de brevet français N° FR 02/01654, comprenant un espace creux central délimité par une structure hélicoïdale (71, 81) composée de spires hélicoïdales non jointives axialement et comportant, à une extrémité axiale, un moyen de préhension.

Les moyens de fixation supérieur (7) et inférieur (8) permettent ainsi de fixer respectivement les plateaux (3, 4) aux parois osseuses sus-jacente et sous-jacente de la colonne vertébrale.

La spirale hélicoïdale peut être insérée en la vissant d'une part dans les vertèbres et d'autre part dans les lumières aménagées entre les piliers du rail.

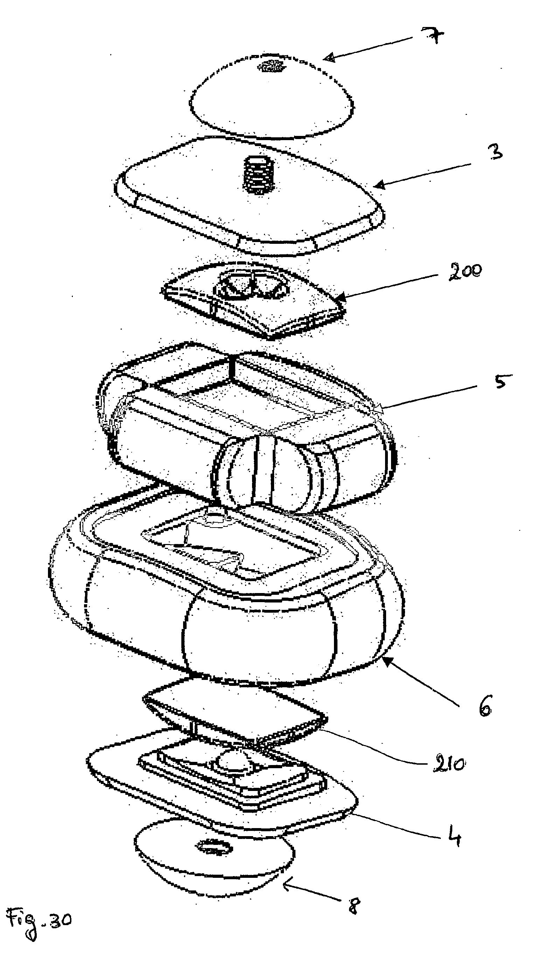

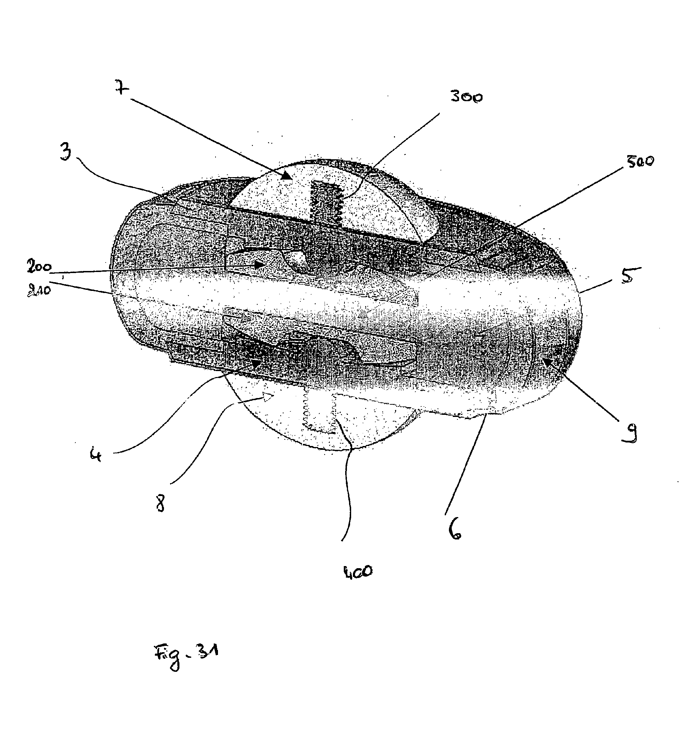

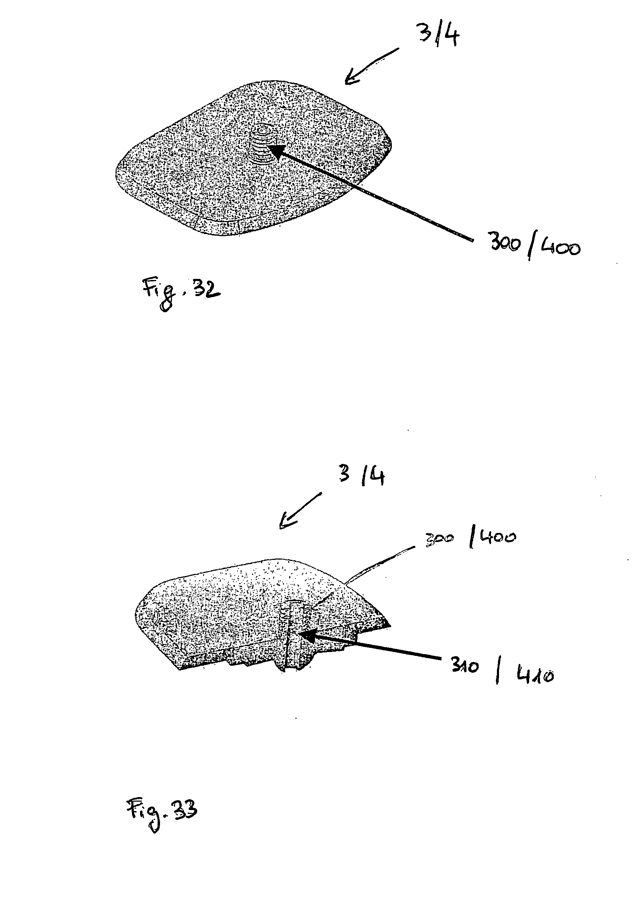

Dans une variante illustrée figures 30 à 33, la prothèse intervertébrale (1) est constituée avantageusement de deux noyaux (200, 210) présentant des rainures en forme de croix de manière à conférer les directions anatomiques.

Dans cette variante, l'enveloppe interne (5) présente une membrane flexible (500) longitudinale s'étendant dans un plan médian de sorte à former deux cavités destinées à recevoir respectivement lesdits noyaux (200, 210).

Afin d'absorber les chocs ainsi que la compression axiale, la membrane (500) de ladite enveloppe interne (5) est constituée de polymère souple.

Les plateaux supérieurs et inférieurs (3, 4) comportent respectivement un orifice traversant (310, 410). Avantageusement, l'orifice traversant (310, 410) est prolongé par une zone tubulaire filetée (300, 400) pour permettre le vissage sur chacun desdits plateaux (3, 4) d'un élément de contact (7, 8). Ces éléments de contact (7, 8) sont destinés à être mis au contact respectivement avec les vertèbres sus-jacente et sous-jacente.

L'ouverture de part et d'autre des plateaux (3, 4) permet ainsi l'injection d'un fluide dans les deux cavités de l'enveloppe interne (5). Avantageusement le fluide injecté dans chacune des cavités de l'enveloppe interne (5) au travers respectivement de chacun desdits plateaux (3, 4) constitue les deux noyaux (200, 210) de la prothèse intervertébrale (1).

Afin de maintenir les éléments constituant la prothèse intervertébrale (1), à savoir les deux noyaux (200, 210), l'enveloppe interne (5), l'enveloppe externe (6) et les plateaux supérieurs et inférieurs (3, 4), tout en maintenant les propriétés d'absorption des chocs de ladite prothèse intervertébrale (1), ces derniers sont avantageusement surmoulés, préalablement au vissage des éléments de contacts sur les plateaux (3, 4), avec un élastomère souple (9).

L'invention est décrite dans ce qui précède à titre d'exemple. Il est entendu que l'homme du métier est à même de réaliser différentes variantes de l'invention sans pour autant sortir du cadre du brevet comme défini par les revendications.

## Revendications

1. - Prothèse intervertébrale (1) du type constituée au moins d'un noyau (2) positionné entre un plateau supérieur (3) et un plateau inférieur (4), ladite prothèse comportant en outre au moins une enveloppe externe (6) lesdits plateaux (3, 4) comportant respectivement des faces intérieures (31, 41) qui permettent un mouvement du noyau (2) à l'intérieur de ladite prothèse, **caractérisé en ce que** ledit noyau (2) est entouré latéralement d'une enveloppe interne (5) souple, ladite enveloppe interne (5) présentant des directions privilégiées de flexibilité, de préférence au nombre de deux et orientées perpendiculairement.

2. - Prothèse intervertébrale (1) selon la revendication 1, **caractérisée en ce que** ladite enveloppe externe (6) est souple.

3. - Prothèse intervertébrale (1) selon la revendication 1 ou la revendication 2, **caractérisée en ce qu'**au moins une face intérieure (31, 41), respectivement desdits plateaux supérieur (3) et/ou inférieur (4) et de préférence les deux faces intérieures (31, 41) des plateaux (3, 4), présente(nt) des moyens de guidage de déplacement dudit noyau (2).

4. - Prothèse intervertébrale (1) selon la revendication 3, **caractérisée en ce que** lesdits moyens de guidage sont orientés selon deux directions perpendiculaires et de préférence dans les mêmes directions que les directions privilégiées de flexibilité de l'enveloppe interne (5).

5. - Prothèse intervertébrale (1) selon la revendication 3 ou la revendication 4, **caractérisée en ce que** lesdits moyens de guidages sont constitués par des plans inclinés (33, 33', 44, 44') dont les bords extérieurs sont orientés vers le noyau (2).

6. - Prothèse intervertébrale (1) selon l'une quelconque des revendications 1 à 5, **caractérisée en ce qu'**au moins une face intérieure (31, 41), respectivement desdits plateaux supérieur (3) et/ou inférieur (4) présente un appendice (30, 40) et **en ce que** ledit noyau (2) comporte en outre respectivement sur sa face supérieure (21) et/ou inférieure (22) deux rainures (23/23', 24/24') orientés selon deux directions perpendiculaires.

7. - Prothèse intervertébrale (1) selon la revendication 6, **caractérisée en ce que** lesdites rainures (23/23', 24/24') sont orientées dans les mêmes directions que les directions privilégiées de flexibilité de l'enveloppe interne (5).

8. - Prothèse intervertébrale (1) selon la revendication 1 ou 2 ou 6 ou 7, **caractérisée en ce qu'**au moins une face intérieure (31, 41), respectivement desdits plateaux supérieur (3) et/ou inférieur (4), et de préférence les deux faces intérieures (31, 41) des plateaux (3, 4), est ou sont plate(s).

9. - Prothèse intervertébrale (1) selon l'une quelconque des revendications 1 à 8, **caractérisée en ce que** ledit noyau (2) présente une forme sensiblement parallélépipédique.

10. - Prothèse intervertébrale (1) selon l'une quelconque des revendications 1 à 9, **caractérisée en ce que** les faces supérieure (21) et inférieure (22) dudit noyau (2) sont arrondies, de préférence selon les directions privilégiées de flexibilité de l'enveloppe interne (5).

11. - Prothèse intervertébrale (1) selon l'une quelconque des revendications 1 à 10, **caractérisée en ce que** ledit noyau (2) présente des bords arrondis.

12. - Prothèse intervertébrale (1) selon l'une quelconque des revendications 1 à 11, **caractérisée en ce que** ledit noyau (2) étant entouré latéralement d'une enveloppe interne (5), ladite enveloppe interne (5) présente en coupe horizontale une forme de croix constituée de quatre bras horizontaux (53, 54, 55, 56) orientés perpendiculairement.

13. - Prothèse intervertébrale (1) selon la revendication 12, **caractérisée en ce que** lesdits bras (53, 54, 55, 56) présentent chacun un trou (57) débouchant sur les faces supérieure (51) et inférieure (52) de ladite enveloppe interne (5).

14. - Prothèse intervertébrale (1) selon la revendication 12 ou la revendication 13, **caractérisée en ce que** les faces centripètes (58) desdits bras (53, 54, 55, 56) sont droites.

15. - Prothèse intervertébrale (1) selon l'une quelconque des revendications 12 à 14, **caractérisée en ce que** les faces centrifuges (59) desdits bras (53, 54, 55, 56) sont arrondies et **en ce que** les faces centripètes de l'enveloppe externe (6) sont arrondies.

16. - Prothèse intervertébrale (1) selon l'une quelconque des revendications 12 à 15, **caractérisée en ce que** ladite enveloppe externe (6) présente des ailettes intérieures (64) destinées à maintenir les bras horizontaux (53, 54, 55, 56) de l'enveloppe interne (5).

17. - Prothèse intervertébrale (1) selon l'une quelconque des revendications 1 à 14, **caractérisée en ce que** l'enveloppe interne (5) et l'enveloppe externe (6) sont venues de matière.

18. - Prothèse intervertébrale (1) selon l'une quelconque des revendications 1 à 17, **caractérisée en ce que** ladite enveloppe externe (6) est ajourée sur ses parois supérieure (61) et/ou inférieure (62), pour le passage des faces extérieures (32, 42), respectivement des plateaux supérieur (3) et inférieur (4).

19. - Prothèse intervertébrale (1) selon la revendication 18, **caractérisée en ce que** ledit plateau ou lesdits plateaux supérieur (3) et/ou inférieur (4) présente/présentent une cavité annulaire (35, 45) adjacente à la face extérieure (32, 42) et **en ce que** ladite paroi ou lesdites parois respectivement supérieure (61) et/ou inférieure (62) présente/présentent un rebord centripète (63, 63') destiné à coopérer avec ladite cavité annulaire (35, 45).

20. - Prothèse intervertébrale (1) selon l'une quelconque des revendications 1 à 19, **caractérisée en ce que** ledit plateau ou lesdits plateaux supérieur (3) et/ou inférieur (4) est ou sont souple/souples.

21. - Prothèse intervertébrale (1) selon l'une quelconque des revendications 1 à 20, **caractérisée en ce que** ledit plateau ou lesdits plateaux supérieur (3) et/ou inférieur (4) présente/présentent sur sa face ou sur leurs faces extérieure/extérieures (32, 42) un rail de fixation (36, 46).

22. - Prothèse intervertébrale (1) selon l'une quelconque des revendications 1 à 21, **caractérisée en ce qu'**elle comporte en outre des moyens de fixations (7, 8), pour la fixation respectivement du plateau ou des plateaux supérieur (3) et/ou inférieur (4).

23. - Prothèse intervertébrale (1) selon l'une quelconque des revendications précédentes, **caractérisée en ce qu'**elle est constituée de deux noyaux (200, 210).

24. - Prothèse intervertébrale (1) selon la revendication 23, **caractérisée en ce que** l'enveloppe interne (5) présente une membrane médiane (500) longitudinale séparant les deux noyaux (200, 210).

25. - Prothèse intervertébrale (1) selon la revendication 24, **caractérisée en ce que** la membrane (500) de ladite enveloppe interne (5) est flexible.

26. - Prothèse intervertébrale (1) selon l'une quelconque des revendications précédentes, **caractérisée en ce qu'**un des plateaux (3, 4) au moins comporte un orifice (310, 410) traversant pour permettre le passage d'un fluide dans ladite enveloppe interne (5).

27. - Prothèse intervertébrale (1) selon la revendication 26, **caractérisée en ce que** l'orifice traversant (310, 410) est prolongé par une zone tubulaire filetée (300, 400) destinée à coopérer avec une zone filetée complémentaire d'un élément de contact (7, 8) destinée à être mis au contact avec une des vertèbres.

28. - Prothèse intervertébrale (1) selon l'une quelconque des revendications précédentes, **caractérisée en ce que** ladite prothèse (1) formée desdits noyaux (2, 200, 210), de l'enveloppe interne (5), de l'enveloppe externe (6) et des plateaux (3, 4) est surmoulée avec un élastomère souple.

29. - Prothèse intervertébrale (1) selon l'une quelconque des revendications précédentes, **caractérisée en ce que** le dit noyau ou les dits noyaux (2, 200, 210) est/sont solide/solides ou liquide/liquides.

## Claims

1. Intervertebral prosthesis (1) of the type made up of at least one core (2) positioned between a top plate (3) and a bottom plate (4), said prosthesis also comprising at least one outer casing (6), said plates (3, 4) respectively comprising inner faces (31, 41) that allow a movement of the core (2) within said prosthesis, **characterised in that** said core (2) is laterally surrounded by a flexible inner casing (5), said inner casing (5) being flexible in preferred directions, preferably two such directions perpendicular to one another.

2. Intervertebral prosthesis (1) according to claim 1, **characterised in that** said outer casing (6) is flexible.

3. Intervertebral prosthesis (1) according to claim 1 or claim 2, **characterised in that** at least one inner face (31, 41) of said top (3) and/or bottom plates (4) respectively, and preferably the two inner faces (31, 41) of the plates (3, 4) comprise(s) means for guiding the movement of said core (2).

4. Intervertebral prosthesis (1) according to claim 3, **characterised in that** said guiding means are positioned according to two perpendicular directions and preferably in the same directions as the preferred directions of flexibility of the inner casing (5).

5. Intervertebral prosthesis (1) according to claim 3 or claim 4, **characterised in that** said guiding means consist of inclined planes (33, 33', 44, 44'), the outer edges of which are directed towards the core (2).

6. Intervertebral prosthesis (1) according to any one of the claims from 1 to 5, **characterised in that** at least one inner face (31, 41) of said top (3) and/or bottom plates (4) respectively comprises an appendix (30, 40) and **in that** said core (2) also respectively comprises, on its top (21) and/or bottom (22) face, two grooves (23/23', 24/24') positioned according to two perpendicular directions.

7. Intervertebral prosthesis (1) according to claim 6, **characterised in that** said grooves (23/23', 24/24') are positioned in the same directions as the preferred directions of flexibility of the inner casing (5).

8. Intervertebral prosthesis (1) according to claim 1 or 2 or 6 or 7, **characterised in that** at least one inner face (31, 41) of said top (3) and/or bottom plates (4) respectively, and preferably the two inner faces (31, 41) of the plates (3, 4) is/are flat.

9. Intervertebral prosthesis (1) according to any one of the claims from 1 to 8, **characterised in that** said core (2) has a substantially parallelepipedal shape.

10. Intervertebral prosthesis (1) according to any one of the claims from 1 to 9, **characterised in that** the top (21) and bottom (22) faces of said core (2) are rounded, preferably according to the preferred directions of flexibility of the inner casing (5).

11. Intervertebral prosthesis (1) according to any one of the claims from 1 to 10, **characterised in that** said core (2) has rounded edges.

12. Intervertebral prosthesis (1) according to any one of the claims from 1 to 11, **characterised in that**, said core (2) being laterally surrounded by an inner casing (5), said inner casing (5) has, in a horizontal cross-section, a cross shape made up of four horizontal arms (53, 54, 55, 56) arranged perpendicularly.

13. Intervertebral prosthesis (1) according to claim 12, **characterised in that** said arms (53, 54, 55, 56) each comprise a hole (57) opening onto the top (51) and bottom (52) faces of said inner casing (5).

14. Intervertebral prosthesis (1) according to claim 12 or claim 13, **characterised in that** the centripetal faces (58) of said arms (53, 54, 55, 56) are straight.

15. Intervertebral prosthesis (1) according to any one of the claims from 12 to 14, **characterised in that** the centrifugal faces (59) of said arms (53, 54, 55, 56) are rounded and **in that** the centripetal faces of the outer casing (6) are rounded.

16. Intervertebral prosthesis (1) according to any one of the claims from 12 to 15, **characterised in that** said outer casing (6) comprises inner fins (64) designed to support the horizontal arms (53, 54, 55, 56) of the inner casing (5).

17. Intervertebral prosthesis (1) according to any one of the claims from 1 to 14, **characterised in that** the inner casing (5) and the outer casing (6) are cast in one piece.

18. Intervertebral prosthesis (1) according to any one of the claims from 1 to 17, **characterised in that** said outer casing (6) has openings on its top (61) and/or bottom (62) walls, for the passage of the outer faces (32, 42) of the top (3) and bottom (4) plates respectively.

19. Intervertebral prosthesis (1) according to claim 18, **characterised in that** said plate or said top (3) and/or bottom (4) plates comprises/comprise an annular cavity (35, 45) adjacent to the outer face (32, 42) and **in that** said wall or said top (61) and/or bottom (62) walls respectively comprises/comprise a centripetal lip (63, 63') designed to cooperate with said annular cavity (35, 45).

20. Intervertebral prosthesis (1) according to any one of the claims from 1 to 19, **characterised in that** said plate or said top (3) and/or bottom (4) plates is/are flexible.

21. Intervertebral prosthesis (1) according to any one of the claims from 1 to 20, **characterised in that** said plate or said top (3) and/or bottom (4) plates comprises/comprise an attachment rail (36, 46) on its/their outer face/faces (32, 42).

22. Intervertebral prosthesis (1) according to any one of the claims from 1 to 21, **characterised in that** it also comprises attachment means (7, 8) for respectively attaching the plate or the top (3) and/or bottom (4) plates.

23. Intervertebral prosthesis (1) according to any one of the preceding claims, **characterised in that** it consists of two cores (200, 201).

24. Intervertebral prosthesis (1) according to claim 23, **characterised in that** the inner casing (5) comprises a longitudinal median membrane (500) separating the two cores (200, 201).

25. Intervertebral prosthesis (1) according to claim 24, **characterised in that** the membrane (500) of said inner casing (5) is flexible.

26. Intervertebral prosthesis (1) according to any one of the preceding claims, **characterised in that** at least one of the plates (3, 4) comprises a through hole (310, 410) allowing the passage of a fluid into said inner casing (5).

27. Intervertebral prosthesis (1) according to claim 26, **characterised in that** the through hole (310, 410) is extended by a threaded tubular area (300, 400) designed to cooperate with a matching threaded area of a contact element (7, 8) designed to be placed in contact with one of the vertebrae.

28. Intervertebral prosthesis (1) according to any one of the preceding claims, **characterised in that** said prosthesis (1) made up of said cores (2, 200, 201), the inner casing (5), the outer casing (6) and the plates (3, 4) is overmoulded with a flexible elastomer.

29. Intervertebral prosthesis (1) according to any one of the preceding claims, **characterised in that** said core or said cores (2, 200, 201) is/are solid or liquid.

## Patentansprüche

1. - Bandscheibenprothese (1) bestehend aus mindestens einem zwischen einer oberen Platte (3) und einer unteren Platte (4) positionierten Kern (2), wobei die genannte Prothese mindestens einen Außenmantel (6), wobei, die genannten Platten (3, 4) jeweils Innenseiten (31, 41) aufweisen, die eine Bewegung des Kerns (2) innerhalb der genannten Prothese ermöglichen, **dadurch gekennzeichnet, dass** der genannte Kern (2) seitlich von einem flexiblen Innenmantel (5) umgeben ist, wobei der genannte Innenmantel (5) bevorzugte Flexibilitätsrichtungen aufweist, vorzugsweise zwei, die senkrecht ausgerichtet sind.

2. Bandscheibenprothese (1) gemäß Anspruch 1, **dadurch gekennzeichnet, dass** der genannte Außenmantel (6) flexibel ist.

3. Bandscheibenprothese (1) gemäß Anspruch 1 oder Anspruch 2, **dadurch gekennzeichnet, dass** mindestens eine Innenseite (31, 41) jeweils der genannten oberen (3) et/oder unteren Platte (4) und vorzugsweise die beiden Innenseiten (31, 41) der Platten (3, 4) Führungsmittel für das Verschieben des genannten Kerns (2) aufweist (aufweisen).

4. Bandscheibenprothese (1) gemäß Anspruch 3, **dadurch gekennzeichnet, dass** die genannten Führungsmittel nach zwei senkrechten und vorzugsweise gleichen Richtungen wie die bevorzugten Flexibilitätsrichtungen des Innenmantels (5) ausgerichtet sind.

5. Bandscheibenprothese (1) gemäß Anspruch 3 oder Anspruch 4, **dadurch gekennzeichnet, dass** die genannten Führungsmittel aus geneigten Ebenen (33, 33', 44, 44') bestehen, deren Außenränder zum Kern (2) hin gerichtet sind.

6. Bandscheibenprothese (1) gemäß einem der Ansprüche 1 bis 5, **dadurch gekennzeichnet, dass** mindestens eine Innenseite (31, 41) jeweils der besagten oberen (3) und/oder unteren Platte (4) einen Ansatz (30, 40) aufweist, und dass der genannte Kern (2) an seiner oberen Seite (21) und/oder unteren Seite (22) zwei nach zwei senkrechten Richtungen gerichtete Rillen (23/23', 24/24') umfaßt.

7. Bandscheibenprothese (1) gemäß Anspruch 6, **dadurch gekennzeichnet, dass** die genannten Rillen (23/23'; 24/24')' in die gleichen Richtungen wie die bevorzugten Flexibilitätsrichtungen des Innenmantels (5) ausgerichtet sind.

8. Bandscheibenprothese (1) gemäß Anspruch 1 oder 2 bzw. 6 oder 7, **dadurch gekennzeichnet, dass** mindestens eine Innenseite (31, 41) jeweils der genannten oberen (3) und/oder unteren Platte (4) und vorzugsweise die beiden Innenseiten (31, 41) der Platten (3, 4) flach ist oder sind.

9. Bandscheibenprothese (1) gemäß einem der Ansprüche 1 bis 8, **dadurch gekennzeichnet, dass** der genannte Kern (2) etwa quaderförmig ist.

10. Bandscheibenprothese (1) gemäß einem der Ansprüche 1 bis 9, **dadurch gekennzeichnet, dass** die obere (21) und untere Seite (22) des genannten Kerns (2) abgerundet sind, vorzugsweise nach den bevorzugten Flexibilitätsrichtungen des Innenmantels (5).

11. Bandscheibenprothese (1) gemäß einem der Ansprüche 1 bis 10, **dadurch gekennzeichnet, dass** der genannte Kern (2) abgerundete Ränder aufweist.

12. Bandscheibenprothese (1) gemäß einem der Ansprüche 1 bis 11, **dadurch gekennzeichnet, dass**, da der genannte Kern (2) seitlich von einem Innenmantel (5) umgeben ist, der genannte Innenmantel (5) im Querschnitt die Form eines Kreuzes aufweist, das aus vier horizontalen, senkrecht ausgerichteten Armen (53, 54, 55, 56) besteht.

13. Bandscheibenprothese (1) gemäß Anspruch 12, **dadurch gekennzeichnet, dass** die genannten Arme (53, 54, 55, 56) jeweils ein an der oberen (51) und unteren Seite (52) des genannten Innenmantels (5) mündendes Loch (57) aufweisen.

14. Bandscheibenprothese (1) gemäß Anspruch 12 oder Anspruch 13, **dadurch gekennzeichnet, dass** die zur Mitte hin gerichteten Seiten (58) der genannten Arme (53, 54, 55, 56) gerade sind.

15. Bandscheibenprothese (1) gemäß einem der Ansprüche 12 bis 14, **dadurch gekennzeichnet, dass** die zur Mitte hin gerichteten Seiten (59) der genannten Arme (53, 54, 55, 56) abgerundet sind, und dass die zur Mitte hin gerichteten Seiten des Außenmantels (6) abgerundet sind.

16. Bandscheibenprothese (1) gemäß einem der Ansprüche 12 bis 15, **dadurch gekennzeichnet, dass** der genannte Außenmantel (6) Innenrippen (64) aufweist, die die horizontalen Arme (53, 54, 55, 56) des Innenmantels (5) festhalten sollen.

17. Bandscheibenprothese (1) gemäß einem der Ansprüche 1 bis 14, **dadurch gekennzeichnet, dass** der Innenmantel (5) und der Außenmantel (6) aus einem Stück bestehen.

18. Bandscheibenprothese (1) gemäß einem der Ansprüche 1 bis 17, **dadurch gekennzeichnet, dass** der genannte Außenmantel (6) an seiner oberen (61) und/oder unteren Wand (62) gelocht ist für den Durchgang der Außenseiten (32, 42) jeweils der oberen (3) und unteren Platte (4).

19. Bandscheibenprothese (1) gemäß Anspruch 18, **dadurch gekennzeichnet, dass** die genannte Platte oder die genannte obere (3) und/oder untere Platte (4) einen ringförmigen, an die Außenseiten (32, 42) grenzenden Hohlraum (35, 45) aufweist, und dass die genannte Wand oder die genannte jeweils obere (61) und/oder untere Wand (62) einen zur Mitte hin gerichteten Rand (63, 63') aufweist, der mit dem genannten ringförmigen Hohlraum (35, 45) zusammenwirken soll oder sollen.

20. Bandscheibenprothese (1) gemäß einem der Ansprüche 1 bis 19, **dadurch gekennzeichnet, dass** die genannte Platte oder die genannte obere (3) und/oder untere Platte (4) flexibel ist oder sind.

21. Bandscheibenprothese (1) gemäß einem der Ansprüche 1 bis 20, **dadurch gekennzeichnet, dass** die genannte Platte oder die genannte obere (3) und/oder untere Platte (4) an ihrer Außenseite oder ihren Außenseiten (32, 42) eine Befestigungsschiene (36, 46) aufweist oder aufweisen.

22. Bandscheibenprothese (1) gemäß einem der Ansprüche 1 bis 21, **dadurch gekennzeichnet, dass** sie ferner Befestigungsmittel (7, 8) für die jeweilige Befestigung der Platte oder der oberen (3) und/oder unteren Platte (4) aufweist.

23. Bandscheibenprothese (1) gemäß einem der vorangehenden Ansprüche, **dadurch gekennzeichnet, dass** sie aus zwei Kernen (200, 210) besteht.

24. Bandscheibenprothese (1) gemäß Anspruch 23, **dadurch gekennzeichnet, dass** der Innenmantel (5) eine mittlere, in Längsrichtung verlaufende Membran (500) aufweist, die die beiden Kerne (200, 210) trennt.

25. Bandscheibenprothese (1) gemäß Anspruch 24, **dadurch gekennzeichnet, dass** die Membran (500) des genannten Innenmantels (5) flexibel ist.

26. Bandscheibenprothese (1) gemäß einem der vorangehenden Ansprüche, **dadurch gekennzeichnet, dass** mindestens eine der Platten (3, 4) eine durchgehende Öffnung (310, 410) umfaßt, um den Durchgang einer Flüssigkeit in den genannten Innenmantel (5) zu ermöglichen.

27. Bandscheibenprothese (1) gemäß Anspruch 26, **dadurch gekennzeichnet, dass** die durchgehende Öffnung (310, 410) von einem rohrförmigen Gewindebereich (300, 400) verlängert wird, der mit einem komplementären Gewindebereich eines Kontaktelements (7, 8) zusammenwirken soll, das mit einem der Wirbel in Kontakt zu setzen ist.

28. Bandscheibenprothese (1) gemäß einem der vorangehenden Ansprüche, **dadurch gekennzeichnet, dass** die genannte, aus den genannten Kernen (2, 200, 210), dem Innenmantel (5), dem Außenmantel (6) und den Platten (3, 4) bestehende Prothese (1) mit einem flexiblen Elastomer konfektioniert ist.

29. Bandscheibenprothese (1) gemäß einem der vorangehenden Ansprüche, **dadurch gekennzeichnet, dass** der genannte Kern oder die genannten Kerne (2, 200, 210) fest oder flüssig ist/sind.
